# EUROPEAN PATENT APPLICATION

(11) **EP 4 331 601 A1**
(43) Date of publication of application: **06.03.2024**
(21) Application number: 22796141.4
(22) Date of filing: 27.04.2022
(51) Int. Cl.: A61K 38/39, A61P 21/00, A23L 33/17, A23K 20/147

(54) **FOOD COMPOSITION AND PHARMACEUTICAL COMPOSITION FOR PREVENTING OR ALLEVIATING SARCOPENIA, CONTAINING LOW-MOLECULAR-WEIGHT COLLAGEN AS ACTIVE INGREDIENT**

(30) Priority: 27.04.2021 KR 20210054546
(71) Applicant: Kyungpook National University Industry-Academic Cooperation Foundation, Daegu 41566 (KR)
(72) Inventor: KWON, Eun-Young, Daegu 41531 (KR); HAN, Youngji, Daegu 41519 (KR); KIM, Ji-Eun, Daegu 41238 (KR)
(74) Representative: Dehns
(86) International application number: PCT/KR2022/006035
(87) International publication number: WO 2022/231309

(57) **Abstract**

The present invention relates to a composition for preventing, alleviating or treating sarcopenia, containing a low-molecular-weight collagen as an active ingredient, and the low-molecular-weight collagen of the present invention inhibits muscle protein breakdown and can exhibit a muscle-strengthening effect through an increase in muscle mass, and thus is expected to be effectively usable for preventing, alleviating or treating sarcopenia.

## Description

### [Technical Field]

The present invention relates to a composition for preventing, alleviating or treating sarcopenia, comprising low-molecular-weight collagen as an active ingredient.

This application claims priority to and the benefit of Korean Patent Application Nos. 10-2021-0054546 and 10-2022-0052000 filed on April 27, 2021 and April 27, 2022, respectively, and all the contents disclosed in the specification and drawings of the applications are incorporated in this application.

### [Background Art]

According to the 2015 UN World Population Prospects (the 2015 revision), it was estimated that the population aged 60 or older, which was 910 million in 2015, will increase to approximately 1.4 billion in 2030 and reach 2.1 billion in 2050. Also, it is expected that the number of super-aged people aged 80 or older will reach approximately 434 million in 2050, and this increase continue for decades to come.

After Korea entered an aging society in 2000 as the population aged 65 or older reached 7.2% of the total population, the National Statistical Office predicted that Korea will enter an aged society in 2015, and will enter a post-aged society as the proportion of the elderly population accounts for 20.8% of the total population in 2026. In particular, it is pointed out as a major problem that Korea is quickly approaching the aged society or the super-aged society in a very short period.

Such an increase in the elderly population may lead to a decrease in productivity due to a decrease in the working population, and a decrease in physical strength due to aging may lower the quality of life of the elderly. Therefore, there is a need for various solutions to solve the above problems.

Meanwhile, the term "aging" refers to a phenomenon in which the body functions of a living organism deteriorate over time. In the case of humans, it is known that the hearing, blood output, lung capacity, and muscle mass decrease by 30%, 45%, 60%, and 50%, respectively, by the age of 80.

A representative physiological change with aging is the loss of muscle mass and strength. It is known that the loss of muscle mass occurs after one's 40s and decreases by approximately 8% per decade until one's 70s. Afterward, is known that muscle mass rapidly decreases after one's 70s, and decreases up to 15% every decade.

Because age-related sarcopenia causes a direct decrease in muscle strength, it is a cause of not only increasing the risk of death due to various body dysfunctions and disabilities, but also increasing the prevalence of metabolic diseases such as high blood pressure, diabetes, arthritis, obesity, cancer, and the like, resulting in reduced metabolism, compromised immunity, and the like. Therefore, research on various therapeutic compositions capable of preventing or treating age-related sarcopenia is being actively conducted.

### [Disclosure]

### [Technical Problem]

The present inventors have made intensive efforts to develop an agent that can effectively treat sarcopenia, particularly age-related sarcopenia, or effectively slow the progression of sarcopenia, and found that low-molecular-weight collagen may efficiently alleviate sarcopenia by increasing muscle mass and strength without any exercise in aged mice having difficulty in exercising. Therefore, the present invention has been completed based on these above facts.

Accordingly, it is an aspect of the present invention to provide a pharmaceutical composition for preventing or treating sarcopenia, comprising low-molecular-weight collagen as an active ingredient.

It is another aspect of the present invention to provide a food composition for preventing or alleviating sarcopenia, comprising low-molecular-weight collagen as an active ingredient.

It is still another aspect of the present invention to provide a feed composition for preventing or alleviating sarcopenia, comprising low-molecular-weight collagen as an active ingredient.

However, the technical objects to be achieved by the present invention are not limited to the above-described technical objects, and other objects which are not mentioned above will be clearly understood from the following detailed description by those skilled in the art to which the present invention pertains.

### [Technical Solution]

To solve the above problems, according to an aspect of the present invention, there is provided a pharmaceutical composition for preventing or treating sarcopenia, comprising low-molecular-weight collagen as an active ingredient.

According to another aspect of the present invention, there is provided a food composition for preventing or alleviating sarcopenia, comprising low-molecular-weight collagen as an active ingredient.

According to still another aspect of the present invention, there is provided a feed composition for preventing or alleviating sarcopenia, comprising low-molecular-weight collagen as an active ingredient.

According to one exemplary embodiment of the present invention, the low-molecular-weight collagen may have an average molecular weight of 1,000 daltons or less, but the present invention is not limited thereto.

According to another exemplary embodiment of the present invention, the low-molecular-weight collagen may comprise 2 to 10% by weight of a collagen tripeptide having a glycine-proline-hydroxyproline amino acid sequence based on the total amount of the low-molecular-weight collagen comprised in the pharmaceutical composition, the food composition, or the feed composition, but the present invention is not limited thereto.

According to still another exemplary embodiment of the present invention, the low-molecular-weight collagen may satisfy one or more of the following characteristics, but the present invention is not limited thereto:
a) increasing the expression of insulin-like growth factor 1 (IGF-1);
b) inhibiting myostatin expression;
c) inhibiting a reduction in muscle strength; and
d) increasing muscle mass.

According to yet another exemplary embodiment of the present invention, the pharmaceutical composition or the food composition may be used for people in their 30s or older who are at risk of developing sarcopenia, but the present invention is not limited thereto.

According to yet another exemplary embodiment of the present invention, the food composition may be a health functional food composition, but the present invention is not limited thereto.

According to yet another aspect of the present invention, there is provided a method of preventing or treating sarcopenia, which comprises: administering the composition comprising low-molecular-weight collagen as an active ingredient to a subject in need thereof.

According to yet another aspect of the present invention, there is provided a use of the composition comprising low-molecular-weight collagen as an active ingredient for the prevention or treatment of sarcopenia.

According to yet another aspect of the present invention, there is provided a use of the low-molecular-weight collagen for the manufacture of a drug for the treatment of sarcopenia.

### [Advantageous Effects]

The low-molecular-weight collagen of the present invention can inhibit the breakdown of muscle proteins and exhibit a muscle-strengthening effect by increasing muscle mass, and thus is expected to be effectively used in the prevention, alleviation or treatment of sarcopenia.

### [Description of Drawings]

FIG. 1A is a diagram showing the results of measuring the thicknesses of the left hind legs of mice in a young control (YC) group, an aging control (AC) group, and a collagen tripeptide (CTP) group (##p < 0.01 vs. CTP, *p < 0.05 vs. YC, **p < 0.01 vs. YC).
FIG. 1B is a diagram showing the results of measuring the whole-body tensions of mice in the YC group, the AC group, and the CTP group (*p < 0.05, **p < 0.01, ***p < 0.001; the same applies hereinafter).
FIG. 1C is a diagram showing the results of measuring the weights of tissues of the quadriceps (left), tibialis anterior (middle), and gastrocnemius (right) of the mice in the YC, AC, and CTP groups. Here, the weight is based on 100 g of body weight.
FIG. 1D is a diagram showing the results of H&E staining (upper left) and Sirius red staining (lower left) on the gastrocnemius tissues of the mice in the YC, AC, and CTP groups, and the results of measuring the muscle cross-sectional area (CSA; upper right) and the collagen area (lower right) of gastrocnemius tissue.
FIG. 2A is a diagram showing the results of confirming the expression of IGF-1 and myostatin in the mice of the YC, AC, and CTP groups through IHC analysis.
FIG. 2B is a diagram showing the results of measuring the protein levels of Akt, PI3K, mTOR, PGC1-α, and phosphorylated variants thereof in the mice of the YC, AC, and CTP groups.
FIG. 3A is a diagram showing the results of measuring the adipose tissue weights of perirenal, mesenteric, retroperitoneal, subcutaneous, interscapular, and epididymal white adipose tissue (WAT) of mice in the YC, AC, and CTP groups and measuring the adipose tissue weight of interscapular brown adipose tissue (BAT).
FIG. 3B is a diagram showing the results of determining the visceral fat, the total adipose tissue weight, the total muscle tissue weight, and the muscle tissue weight per 100 g of body weight in the mice of the YC, AC, and CTP groups.
FIG. 3C is a diagram confirming the lipid profiles of the gastrocnemius (quad) in the mice of the YC, AC, and CTP groups

### [Best Mode]

The present inventors have made intensive efforts to develop an agent that can effectively treat sarcopenia, particularly age-related sarcopenia, or effectively slow the progression of sarcopenia, and found that low-molecular-weight collagen may efficiently alleviate sarcopenia by increasing muscle mass and strength without any exercise in aged mice having difficulty in exercising. Therefore, the present invention has been completed based on the above facts.

Hereinafter, the present invention will be described in detail.

The present invention relates to a pharmaceutical composition for preventing or treating sarcopenia, comprising low-molecular-weight collagen as an active ingredient.

In this specification, the term "low-molecular-weight collagen" refers to a low-molecular-weight peptide obtained by enzymatic degradation of collagen derived from fish skin, pork skin, and the like. In general, collagen may be divided into high-molecular-weight collagen and low-molecular-weight collagen according to its molecular weight. Here, high-molecular-weight collagen generally refers to collagen having a molecular weight of 3,000 daltons (Da) or more, and low-molecular-weight collagen refers to collagen having a molecular weight of 1,000 Da or less. According to one embodiment of the present invention, the low-molecular-weight collagen may comprise 3.2% or more of glycine-proline-hydroxyproline (GPH) and 30% or more of collagen tripeptides, but the present invention is not limited thereto.

Because the low-molecular-weight collagen has a smaller molecular weight (1,000 Da or less) than high-molecular-weight collagen, which has low *in vivo* bioavailability because it is not easily decomposed in the body, the low-molecular-weight collagen has high *in vivo* bioavailability in the skin, bone, muscle, hair, and the like because it has excellent body absorption and an excellent absorption rate

In the present invention, the low-molecular-weight collagen may be obtained by the enzymatic breakdown of fish skin-derived collagen. Preferably, the low-molecular-weight collagen may be obtained by enzymatically breaking down fish skin-derived collagen with a non-pathogenic *Bacillus* collagenase-type protease.

In the present invention, the low-molecular-weight collagen may have an average molecular weight of 1,000 daltons or less, 900 daltons or less, 800 daltons or less, 700 daltons or less, 600 daltons or less, 500 daltons or less, 400 daltons or less, or 300 daltons or less, and an average molecular weight of 200 to 700 daltons, 200 to 650 daltons, 200 to 600 daltons, 200 to 550 daltons, 200 to 530 daltons, 200 to 500 daltons, 200 to 450 daltons, 200 to 400 daltons, 250 to 400 daltons, 250 to 450 daltons, 250 to 500 daltons, 250 to 530 daltons, 250 to 550 daltons, 250 to 600 daltons, 300 to 600 daltons, 300 to 550 daltons, 300 to 530 daltons, 300 to 500 daltons, 300 to 450 daltons, 300 to 400 daltons, 350 to 400 daltons, 350 to 450 daltons, 350 to 500 daltons, 400 to 500 daltons, 450 to 500 daltons, or 500 daltons, but the present invention is not limited thereto.

In the present invention, the low-molecular-weight collagen may comprise 2 to 10% by weight, 2 to 9% by weight, 2 to 8% by weight, 2 to 7% by weight, 2 to 6% by weight, 3 to 9% by weight, 3 to 8% by weight, 3 to 7% by weight, 3 to 6% by weight, 3 to 5% by weight, 3 to 4% by weight, 3.2 to 7% by weight, 3.2 to 6% by weight, 3.2 to 5% by weight, or 3.2% by weight or more of a collagen tripeptide having a glycine-proline-hydroxyproline (GPH) amino acid sequence, based on the total amount of the low-molecular-weight collagen comprised in the composition according to the present invention, but the present invention is not limited thereto.

The term "collagen tripeptide having a glycine-proline-hydroxyproline (GPH) amino acid sequence" refers to a tripeptide in which three amino acids, glycine, proline, and hydroxyproline, are repeatedly linked in a helical structure, and thus may be absorbed before a digestive process in the same manner as in the *in vivo* collagen structure. Therefore, sarcopenia may be prevented, alleviated or treated with a small amount of the low-molecular-weight collagen because the GPH amino acid sequence has a high *in vivo* absorption rate.

In the present invention, the low-molecular-weight collagen may satisfy one or more of the following characteristics, but the present invention is not limited thereto:
a) increasing the expression of insulin-like growth factor 1 (IGF-1);
b) inhibiting myostatin expression;
c) inhibiting a reduction in muscle strength; and
d) increasing muscle mass.

In the present invention, the term "insulin-like growth factor 1 (IGF-1)" is insulin-like growth factor 1, which is also referred to as somatomedin-C, and is a hormone having a similar molecular structure to insulin, which plays an important role in the promotion of cell proliferation during childhood development, and also plays an important role in the development and maintenance of muscles and bones. In particular, because IGF-1 may stimulate the expression of mTOR, which stimulates the synthesis of muscle protein, by activating a PI3k/Akt signaling pathway, a decrease in IGF-1 expression with aging affects a reduction in muscle mass.

Also, when Akt is phosphorylated, it suppresses the expression of FoxO3, which is expressed in skeletal muscle to break down protein and is an important regulator of atrogin1 and MuRF-1 expression, which plays a role in regulating myogenesis via a myostatin signal, thereby inhibiting the breakdown of muscle proteins.

In the present invention, the term "myostatin" is a protein that regulates muscle growth, and is also growth and differentiation factor-8 (GDF-8) that belongs to the transforming growth factor-β (TGF-β) family. Myostatin is known to directly bind to activin receptor type 2 and affect the Smad signaling mechanism. In this case, when the activity of myostatin is inhibited, myostatin plays an important role in the alleviation of various metabolic diseases because muscle differentiation is promoted.

In this specification, the term "increasing muscle strength" or "inhibiting a reduction in muscle strength" refers to a phenomenon in which overall muscle strength increases due to an increase in skeletal muscle mass or an improvement in muscle function, and does not refer to an effect limited to a group of patients with a specific disease.

In the present specification, the term "increase in muscle mass" refers to an action of improving the growth of muscle among the body components. In this case, muscle mass may be increased through physical exercise and improved endurance, and may also be increased by administering a substance having a muscle growth effect into the body. The type of muscle is not limited.

In this specification, the term "sarcopenia" refers to a degenerative disease in which muscle mass and strength are reduced, that is, a disease in which muscle mass abnormally and rapidly decreases unlike the muscle loss that occurs in a normal aging process. Such sarcopenia is divided into primary sarcopenia caused by aging and secondary sarcopenia caused by various causes. The causes of secondary sarcopenia comprise malnutrition, decreased activity, organ failure, drugs, inflammation, malignant diseases, or endocrine/metabolic diseases. According to one embodiment of the present invention, sarcopenia may be primary sarcopenia caused by aging, but the present invention is not limited thereto.

The term "primary sarcopenia caused by aging" or "age-related sarcopenia" refers to a disease in which the size and number of muscle fibers decrease with aging, which results in a decrease in muscle density and gradually weakened function, thereby reducing muscle mass.

In the present invention, the composition may be used for people in their 30s or older, who are suspected of having sarcopenia, preferably people in their 40s or older, who are suspected of having sarcopenia, more preferably people in their 50s or older, who are suspected of having sarcopenia, and even more preferably people in their 60s or older, who are suspected of having sarcopenia. In this case, the composition may be used for all people who are suspected of having sarcopenia without age restrictions.

In the present specification, the term "active ingredient" refers to a component that may exhibit the desired activity alone or in combination with a carrier having no activity itself.

In the present invention, the term "pharmaceutical composition" refers to a composition prepared for the purpose of preventing or treating a disease, and may be separately formulated and used in various forms according to conventional methods. For example, the pharmaceutical composition may be formulated into oral formulations such as powders, granules, tablets, capsules, suspensions, emulsions, syrups, and the like, and may also be formulated and used in the form of external preparations, suppositories and sterile injection solutions.

The pharmaceutical composition according to the present invention may further include suitable carriers, excipients and diluents commonly used in the preparation of pharmaceutical compositions. For example, the excipient may include one or more selected from the group consisting of a diluent, a binder, a disintegrant, a lubricant, an adsorbent, a moisturizer, a film-coating material, and a controlled-release additive.

The pharmaceutical compositions according to the present invention may be separately formulated and used in the form of external preparations, such as powders, granules, sustained-release granules, enteric granules, solutions, eye drops, elixirs, emulsions, suspensions, spirits, troches, perfumes, limonades, tablets, sustained-release tablets, enteric tablets, sublingual tablets, hard capsules, soft capsules, sustained-release capsules, enteric capsules, pills, tinctures, soft extracts, dry extracts, fluid extracts, injections, capsules, perfusates, plasters, lotions, pastes, sprays, inhalants, patches, sterile injection solutions, aerosols, or the like, according to conventional methods. Also, the external preparations may have a formulation such as a cream, a gel, a patch, a spray, an ointment, a plaster, a lotion, a liniment, a paste, a cataplasma, or the like.

The carriers, excipients, and diluents that may be included in the pharmaceutical composition according to the present invention include lactose, dextrose, sucrose, oligosaccharides, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil.

When formulated, the pharmaceutical composition is prepared using commonly used diluents or excipients such as fillers, extenders, binders, wetting agents, disintegrants, surfactants, and the like.

As the additives for tablets, powders, granules, capsules, pills, and troches according to the present invention, excipients such as corn starch, potato starch, wheat starch, lactose, sucrose, glucose, fructose, di-mannitol, precipitated calcium carbonate, synthetic aluminum silicate, calcium monohydrogen phosphate, calcium sulfate, sodium chloride, sodium hydrogen carbonate, purified lanolin, microcrystalline cellulose, dextrin, sodium alginate, methyl cellulose, sodium carboxymethyl cellulose, kaolin, urea, colloidal silica gel, hydroxypropyl starch, hydroxypropylmethyl cellulose (HPMC) 1928, HPMC 2208, HPMC 2906, HPMC 2910, propylene glycol, casein, calcium lactate, Primojel, and the like; binders such as gelatin, gum arabic, ethanol, agar powder, cellulose acetate phthalate, carboxymethyl cellulose, calcium carboxymethyl cellulose, glucose, purified water, sodium caseinate, glycerin, stearic acid, sodium carboxymethyl cellulose, sodium methyl cellulose, methyl cellulose, microcrystalline cellulose, dextrin, hydroxycellulose, hydroxypropyl starch, hydroxymethyl cellulose, purified shellac, gelatinized starch, hydroxypropyl cellulose, hydroxypropyl methyl cellulose, polyvinyl alcohol, polyvinyl pyrrolidone, and the like may be used. Also, disintegrants such as hydroxypropyl methyl cellulose, corn starch, agar powder, methyl cellulose, bentonite, hydroxypropyl starch, sodium carboxymethyl cellulose, sodium alginate, calcium carboxymethyl cellulose, calcium citrate, sodium lauryl sulfate, silicic anhydrides, 1-hydroxy propyl cellulose, dextran, ion exchange resins, polyvinyl acetate, formaldehyde-treated casein and gelatin, alginic acid, amylose, guar gum, sodium hydrogen carbonate, polyvinyl pyrrolidone, calcium phosphate, gelatinized starch, gum arabic, amylopectin, pectin, sodium polyphosphate, ethyl cellulose, sucrose, magnesium aluminum silicate, a di-sorbitol solution, light silicic anhydrides, and the like; lubricants such as calcium stearate, magnesium stearate, stearic acid, hydrogenated vegetable oil, talc, lycopodium, kaolin, petroleum jelly, sodium stearate, cacao butter, sodium salicylate, magnesium salicylate, polyethylene glycol (PEG) 4000, PEG 6000, liquid paraffin, hydrogenated soybean oil (Lubri wax), aluminum stearate, zinc stearate, sodium lauryl sulfate, magnesium oxide, Macrogol, synthetic aluminum silicate, silicic anhydrides, higher fatty acids, higher alcohols, silicone oil, paraffin oil, polyethylene glycol fatty acid ethers, starch, sodium chloride, sodium acetate, sodium oleate, dl-leucine, light silicic anhydrides, and the like may be used.

As the additives for solutions according to the present invention, water, dilute hydrochloric acid, dilute sulfuric acid, sodium citrate, sucrose monostearates, polyoxyethylene sorbitol fatty acid esters (Tween esters), polyoxyethylene monoalkyl ethers, lanolin ethers, lanolin esters, acetic acid, hydrochloric acid, aqueous ammonia, ammonium carbonate, potassium hydroxide, sodium hydroxide, prolamine, polyvinyl pyrrolidone, ethyl cellulose, sodium carboxymethyl cellulose, and the like may be used.

In the syrup according to the present invention, a solution of sucrose, other sugars, or a sweetener may be used. When necessary, aromatics, coloring agents, preservatives, stabilizers, suspending agents, emulsifiers, thickeners, and the like may be used.

Purified water may be used in the emulsions according to the present invention, and emulsifiers, preservatives, stabilizing agents, fragrances, and the like may be used when necessary.

As the suspending agents according to the present invention, suspending agents such as acacia, tragacanth, methyl cellulose, carboxymethyl cellulose, sodium carboxymethyl cellulose, microcrystalline cellulose, sodium alginate, hydroxypropyl methyl cellulose (HPMC), HPMC 1828, HPMC 2906, HPMC 2910, and the like may be used. When necessary, surfactants, preservatives, stabilizers, coloring agents, and fragrances may be used.

The injections according to the present invention may include solvents such as distilled water for injection, a 0.9% sodium chloride injection, a dextrose injection, a dextrose + sodium chloride injection, PEG, lactated Ringer's solution, ethanol, propylene glycol, nonvolatile sesame oil, cottonseed oil, peanut oil, soybean oil, corn oil, ethyl oleate, isopropyl myristate, benzene benzoate, and the like; solubilizing aids such as sodium benzoate, sodium salicylate, sodium acetate, urea, urethane, monoethylacetamide, butazolidine, propylene glycol, Tweens, nicotinamide, hexamine, dimethylacetamide, and the like; buffers such as weak acids and salts thereof (acetic acid and sodium acetate), weak bases and salts thereof (ammonia and ammonium acetate), organic compounds, proteins, albumin, peptones, gums, and the like; isotonic agents such as sodium chloride; stabilizers such as sodium bisulfite (NaHSO₃), carbon dioxide gas, sodium metabisulfite (Na₂S₂O₅), sodium sulfite (Na₂SO₃), nitrogen gas (N₂), ethylene diamine tetraacetic acid, and the like; antioxidants such as 0.1% sodium bisulfide, sodium formaldehyde sulfoxylate, thiourea, ethylene diamine disodium tetraacetate, acetone sodium bisulfite, and the like; analgesics such as benzyl alcohol, chlorobutanol, procaine hydrochloride, glucose, calcium gluconate, and the like; and suspending agents such as CMC sodium, sodium alginate, Tween 80, aluminum monostearate, and the like.

As the suppositories according to the present invention, bases such as cacao butter, lanolin, Witepsol, polyethylene glycol, glycerogelatin, methyl cellulose, carboxymethyl cellulose, a mixture of stearic acid and oleic acid, Subanal, cottonseed oil, peanut oil, palm oil, cacao butter + cholesterol, lecithin, Lanette Wax, glycerol monostearate, Tween or Span, Imhausen, monolene (propylene glycol monostearate), glycerin, Adeps solidus, Buytyrum Tego-G, Cebes Pharma 16, Hexalide Base 95, Cotomar, Hydrokote SP, S-70-XXA, S-70-XX75 (S-70-XX95), Hydrokote 25, Hydrokote 711, Idropostal, Massa estrarium (A, AS, B, C, D, E, I, T), Massa-MF, Marsupol, Marsupol-15, Neosupostal-N, Paramound-B, Suposiro (OSI, OSIX, A, B, C, D, H, L), suppository type IV (AB, B, A, BC, BBG, E, BGF, C, D, 299), Supostal (N, Es), Wecobi (W, R, S, M, Fs), Tegester triglyceride bases (TG-95, MA, 57), and the like may be used.

Solid formulations for oral administration include tablets, pills, powders, granules, capsules, and the like, and such solid formulations may be prepared by mixing an extract with at least one excipient such as starch, calcium carbonate, sucrose or lactose, gelatin, and the like. In addition to simple excipients, lubricants such as magnesium stearate and talc may also be used.

Liquid formulations for oral administration include suspensions, internal solutions, emulsions, syrups, and the like. In this case, various excipients such as wetting agents, sweeteners, flavoring agents, preservatives, and the like may be included in addition to commonly used simple diluents such as water, liquid paraffin, and the like. Formulations for parenteral administration include sterile aqueous solutions, non-aqueous solvents, suspensions, emulsions, freeze-dried formulations, and suppositories. Propylene glycol, polyethylene glycol, vegetable oils such as olive oil, injectable esters such as ethyl oleate, and the like may be used as the non-aqueous solvents and suspending agents.

The pharmaceutical composition according to the present invention may be administered in a pharmaceutically effective amount. In the present invention, the "pharmaceutically effective amount" refers to an amount sufficient to treat a disease with a reasonable benefit/risk ratio applicable to medical treatment, and a level of the effective amount may be determined according to the type of a patient's disease, the severity of the disease, the activity of a drug, the sensitivity to the drug, the time of administration, the route of administration, and the excretion rate, the duration of treatment, factors including drugs used concurrently, and other factors well known in the medical field.

The pharmaceutical composition according to the present invention may be administered as an individual therapeutic agent or in combination with other therapeutic agents, and may be administered sequentially or concurrently with a conventional therapeutic agent and may be administered once or multiple times. It is important to administer the pharmaceutical composition according to the present invention in an amount sufficient to obtain the maximum effect with the minimum amount without side effects in consideration of all the above-mentioned factors, and such an amount may be easily determined by a person having ordinary skill in the technical field to which the present invention pertains.

The pharmaceutical composition according to the present invention may be administered to a subject through various routes of administration. All modes of administration may be expected, and may, for example, include oral administration, subcutaneous injection, intraperitoneal administration, intravenous injection, intramuscular injection, injection into the paraspinal space (intrathecal), sublingual administration, buccal administration, rectal insertion, vaginal insertion, intraocular administration, auricular administration, intranasal administration, inhalation, spraying through the mouth or nose, intradermal administration, transdermal administration, and the like.

The pharmaceutical composition of the present invention may be determined according to the type of drug as an active ingredient together with various related factors such as the type of disease to be treated, the route of administration, the age, gender, and weight of a patient, the severity of a disease, and the like.

Also, the present invention provides a method of preventing or treating sarcopenia, which comprises: administering the composition comprising low-molecular-weight collagen as an active ingredient to a subject in need thereof.

In addition, the present invention provides a use of the composition comprising low-molecular-weight collagen as an active ingredient for the prevention or treatment of sarcopenia.

Additionally, the present invention provides a use of the low-molecular-weight collagen for the manufacture of a drug for the treatment of sarcopenia.

In the present invention, the term "subject" refers to a subject in need of treatment of a disease, and, more specifically, a mammal such as a human or non-human primate, a mouse, a rat, a dog, a cat, a horse, a cow, and the like.

In the present invention, the term "administration" refers to an action of providing a predetermined composition of the present invention to a subject using any suitable method.

In the present invention, the term "prevention" refers to all actions of suppressing or delaying the onset of a target disease, the term "treatment" refers to all actions of improving or beneficially changing the target disease and the resulting metabolic abnormalities through the administration of the pharmaceutical composition according to the present invention, and the term "alleviation" refers to all actions of reducing parameters associated with the target disease, for example, the severity of a symptom, through the administration of the composition according to the present invention.

Further, the present invention provides a food composition for preventing or alleviating sarcopenia, which comprises low-molecular-weight collagen as an active ingredient.

The low-molecular-weight collagen comprised in the food composition of the present invention is as described above.

When the low-molecular-weight collagen of the present invention is used as a food additive, the low-molecular-weight collagen may be added as it is, or used together with other foods or food ingredients. In this case, the low-molecular-weight collagen of the present invention may be used appropriately according to conventional methods. The mixing amount of the active ingredient may be appropriately determined according to the purpose of use (prevention, health or therapeutic treatment). In general, when foods or beverages are prepared, the low-molecular-weight collagen of the present invention may be added in an amount of 15% by weight or less, or 10% by weight or less based on the total weight of the raw material. However, in the case of long-term intake for the purpose of health and hygiene or for the purpose of health control, the amount may be less than the above range. Because the active ingredient has no problems in terms of safety, the active ingredient may be used in an amount greater than the above range.

There is no particular limitation on the type of food. Examples of foods to which the above substance may be added may include meat, sausage, bread, chocolate, candies, snacks, confectioneries, pizza, ramen, other noodles, gum, dairy products including ice cream, various soups, beverages, tea, drinks, alcoholic beverages, vitamin complexes, and the like, and include all health functional foods in the usual sense.

A health beverage composition according to the present invention may contain various flavoring agents, natural carbohydrates, and the like as additional components, as in conventional beverages. The above-described natural carbohydrates include monosaccharides such as glucose and fructose, disaccharides such as maltose and sucrose, polysaccharides such as dextrin and cyclodextrin, and sugar alcohols such as xylitol, sorbitol, erythritol, and the like. As sweeteners, natural sweeteners such as thaumatin and stevia extracts, synthetic sweeteners such as saccharin and aspartame, and the like may be used. The proportion of the natural carbohydrates per 100 mL of the composition of the present invention may be generally in a range of approximately 0.01 to 0.20 g, or 0.04 to 0.10 g.

In addition to the above-described substances, the composition of the present invention may contain various nutrients, vitamins, electrolytes, flavors, colorants, pectic acid and salts thereof, alginic acid and salts thereof, organic acids, protective colloidal thickeners, pH adjusters, stabilizing agents, preservatives, glycerin, alcohol, carbonating agents used for carbonated beverages, and the like. In addition, the composition of the present invention may contain fruit flesh for preparing natural fruit juice, fruit juice beverages, and vegetable beverages. These ingredients may be used individually or in combination. The proportion of these additives is not highly critical, but may generally be selected in a range of 0.01 to 0.20 parts by weight based on 100 parts by weight of the composition of the present invention.

In the present invention, the food composition may be a health functional food composition, but the present invention is not limited thereto.

In this specification, the term "health functional food" is the same term as a food for special health use (FoSHU), and refers to a processed food with high medicinal and medical effects to efficiently exhibit bio-regulatory functions in addition to nutrient supply. In this case, the food may be prepared in various forms such as tablets, capsules, powders, granules, liquids, pills, and the like in order to obtain useful effects for preventing or alleviating sarcopenia.

The health functional food of the present invention may be prepared by a method commonly used in the art, and may be prepared by adding raw materials and components commonly added in the art during the preparation. Also, unlike general drugs, the health functional food of the present invention has an advantage in that it has no side effects that may occur when taking a drug for a long time because food is used as a raw material, and may have excellent portability.

Further, the present invention provides a feed composition for preventing or alleviating sarcopenia, comprising low-molecular-weight collagen as an active ingredient.

The low-molecular-weight collagen comprised in the feed composition of the present invention is as described above.

Feed including the low-molecular-weight collagen according to the present invention as the active ingredient may be prepared into various forms of feed known in the art, and may preferably comprise concentrated feed, coarse feed, or specialty feed.

The concentrated feed comprises seeds and fruits including grains such as wheat, oats, corn, and the like; bran including rice bran, wheat bran, barley bran, and the like as by-products remaining after refining grain; dregs such as by-products obtained by collecting oil from soybeans, fluids, sesame seeds, linseed, coco palm, and the like, and residual starchy components, which are the main components of starch residue remaining after removing starch from sweet potatoes, potatoes, and the like; fish solubles obtained by concentrating fresh liquids obtained from fish meal, fish residue, and fresh fish; meat meal; blood meal; feather meal; skim milk powder; animal feed such as dried whey obtained by drying whey which is a residual solution when cheese and casein are prepared from milk and skim milk, respectively; yeast; chlorella; seaweed; and the like.

The coarse feed comprises fresh grass feed such as wild grass, herbage, green cuttings, and the like; root vegetables such as turnips for feed, beets for feed, rutabagas which are a type of turnip, and the like; silage, which is storage feed obtained by filling fresh grass, green crops, grains, and the like in a silo and fermenting with lactic acid; dried grass obtained by cutting and drying wild grass and herbage; straw of crops for breeding livestock; leaves of leguminous plants; and the like.

The speciality feed comprises mineral feeds such as oyster shells, halite, and the like; urea feed such as urea or derivatives thereof (such as diureide isobutene), and the like; feed additives which are added in a small amount into mixed feed in order to supplement ingredients which may be lacking when only mixing natural feed ingredients or to increase the shelf life of feed; dietary supplements, and the like.

Meanwhile, the feed composition according to the present invention may comprise a feed additive, or may be a feed additive.

In the present invention, the term "feed additive" refers to a substance that is added to feed for various purposes, such as supplementation of nutrients and prevention of weight loss, enhancement of digestibility of feed fiber, improvement of oil quality, prevention of reproductive disorders and improvement of fertility rate, and prevention of high-temperature stress in summer. The feed additive of the present invention corresponds to supplementary feed under the Feed Management Act, and may further include inorganic preparations such as sodium bicarbonate, bentonite, magnesium oxide, complex minerals, and the like; mineral preparations, for example, trace minerals such as zinc, copper, cobalt, selenium, and the like; vitamin preparations such as carotene, vitamin E, vitamin A, vitamin D, nicotinic acid, a vitamin B complex, and the like; protective amino acid preparations such as methionine, lysine, and the like; protective fatty acid preparations such as calcium salts of fatty acids and the like; probiotic preparations (a lactic preparation); yeast cultures; live bacteria such as mold-fermented products, yeast preparations, and the like.

The feed composition according to the present invention may be applied to any subject without any particular limitation as long as it is intended for the purpose of preventing, alleviating or treating sarcopenia. The subject represents a mammal including animals, for example, non-primates (e.g., cows, pigs, horses, cats, dogs, rats and mice) and primates (e.g., monkeys (such as cynomolgus monkeys) and chimpanzees).

In still another exemplary embodiment, the subject may be a companion animal (e.g., a dog, a cat, or the like).

As the standard of living improves and the perception of companion animals changes, interest in functional feed capable of improving the health of companion animals is also increasing. Although there are some differences depending on the species or living environment, companion animals also have a problem in that muscle mass and strength decrease due to a decrease in amount of exercise as they become older. Therefore, the feed composition according to the present invention may be applied to prevent or alleviate sarcopenia.

The dose of the feed composition according to the present invention may depend on various factors such as the species, size, weight, and age of the subject. In principle, a typical dose may be in a range of 0.001 to 10 g per subject/day.

### [Mode for Invention]

Hereinafter, preferred experimental examples and embodiments of the present invention are presented in order to aid in understanding the present invention. However, it should be understood that the following experimental examples and embodiments are given by way of illustration only to more easily understand the present invention, and are not intended to limit the present invention.

### [EXPERIMENTAL EXAMPLES]

### Experimental Example 1: Experimental materials

The low-molecular-weight collagen (approximately 500 Da, CTP) used in this experiment is a protein that is extracted from catfish fish skin gelatin and digested with a non-pathogenic *Bacillus* collagenase-type protease (Amicogen Inc., Jinju, South Korea). Typical collagen hydrolysates contain less than 1% collagen tripeptides having an average molecular weight of 2,000 Da or more and do not contain Gly-Pro-Hyp (GPH), whereas the low-molecular-weight collagen includes 3.2% or more of GPH and 30% or more of collagen tripeptides.

The collagen tripeptide refers to small collagen (usually having a molecular weight of 200 to 500 Da) in which three amino acids (glycine (Gly)-x-y) are linked, and may be easily absorbed into the body due to its small molecular weight.

### Experimental Example 2: Animal experiment

C57BL/6J mice (n = 12, 12 months old) and 6 male C57BL/6J mice (8 weeks old) were purchased from JA BIO (Suwon, South Korea). The animals were raised at a temperature (20 - 23 °C) in a light-controlled space (12 hour light/dark cycle). After the purchase, the animals were fed commercial unrefined pelleted feed for 7 days. The twelve-month-old age mice were randomly divided into two groups, and fed each experimental diet for 12 weeks, as shown in Table 1. Specifically, the mice were divided into an aging control (AC) group (n = 6, a negative control, American Institute of Nutrition AIN93G Semisynthetic Diet), and a collagen tripeptide (CTP) group (n = 6, AIN93G + 0.2% CTP). A young control (YC) group (n = 6, YC, American Institute of Nutrition AIN93G Semisynthetic Diet) was also fed the experimental diet for 12 weeks as shown in Table 1.

Free access to distilled water was provided during the experiment, the feed intake was recorded daily, and body weight was measured weekly.

**[Table 1]**

| **Ingredient(g)** | **YC (AIN-93G)** | **AC (AIN-93G)** | **AC +0.2% CTP** |
|---|---|---|---|
| **Casein** | 200 | 200 | 200 |
| **Corn Starch** | 397.5 | 397.5 | 397.5 |
| **Sucrose** | 100 | 100 | 100 |
| **Maltodextrin** | 132 | 132 | 132 |
| **Cellulose** | 50 | 50 | 48 |
| **Soybean Oil** | 70 | 70 | 70 |
| **Mineral Mix** | 25 | 25 | 25 |
| **Vitamin Mix** | 10 | 10 | 10 |
| **TBHQ, antioxidant** | 0.014 | 0.014 | 0.014 |
| **L-Cystine** | 3 | 3 | 3 |
| **Cholin Bitartrate** | 2.5 | 2.5 | 2.5 |
| **CTP** | - | - | 2 |
| **Total (g)** | 1000 | 1000 | 1000 |
| **Calorie (Kcal/g)** | 3948 | 3948 | 3956 |

| | | | |
|---|---|---|---|
| *Mineral Mix (AIN-93G) (gram/kg): Calcium Carbonate 357, Monopotassium Phosphate 196%, Potassium Citrate Monohydrate 70.78, Sodium Chloride 74, Potassium Sulfate 46.6, Magnesium Oxide 24, Ferric Citrate 6.06, Zinc Carbonate 1,65, Manganese Carbonate 0.63, Copper Carbonate 0.3, Potassium Iodate 0.01%, Sodium Selenate, Anhydrous 0.0103, Anmonium Molybdate.4H2O 0.00795, Sodium Metasilicate 9H2O 1.45, Chromium Potasium Sulfate.12H2O 0.275, Lithium Chloride 0.0174, Boric Acid 0.08145, Sodium Fluoride 0.0635, Nickel Carbonate 0.0318, Ammonium Vanadate 0.0066, Powdered Sugar 221 †Vitamin mix (AIN-93G) (gram/kg): Nicotinic Acid 3.00, D-Calcium Pantothenate 1.60, Pyridoxine HCl 0.70, Thiamine HCl 0.60, Riboflavin 0.60, Folic Acid 0.20, D-Biotin 0.02, Vitamin B12 (0.1% triturated in mannitol) 2.50, a-Tocopherol Powder (250U/gm) 30.00, Vitamin A Palmitate (250,000 U/gm) 1.60, Vitamin D3 (400,000 U/gm) 0.25, Phylloquinone 0.075, Powdered Sucrose 959.655 | | | |

### Experimental Example 3: Measurement of whole-body tension

The whole-body tensions of the mice were measured and evaluated using a digital force gauge (FGJN.FGP Series, JM Instruments Corp., Seoul, South Korea). To measure the whole-body tension, after 5 days of practice, the tension of each mouse was measured every day, and the average maximum tension value was calculated in grams (g).

### Experimental Example 4: Measurement of left hind leg thickness

The thicknesses of left hind legs were measured using Bluetec digital calipers (BD500; BLUETEC, Seoul, South Korea) every 4 weeks.

### Experimental Example 5: Histopathological analysis and immunohistochemical analysis

Gastrocnemius muscle tissues were removed from the mice, and fixed in a 10% formalin buffer. The fixed gastrocnemius muscle tissues were routinely processed for paraffin embedding, prepared into 4 µm-sized sections, and stained with H&E and Sirius red. Thereafter, immunohistochemistry was performed for insulin-like growth factor 1 (IGF-1) and myostatin. The slides were observed using a Moti slide scanner at 20x magnification. Histopathological analysis and immunohistochemical analysis were performed based on previously known methods.

### Experimental Example 6: Muscle lipid profile analysis

After gastrocnemius muscle lipids were extracted, the dried lipid residue was dissolved in 1 mL of ethanol for the measurement of triglyceride (TG), total cholesterol (TC), and fatty acid (FA). For emulsification, a solution of Triton X-100 and sodium cholate was added to 200 µL of the dissolved lipid solution. Enzymatic analysis of TG and TC was performed using a commercial kit (Asan Pharm Co., Seoul, Republic of Korea). FA was measured using an enzymatic assay (Abcam, Cambridge, USA).

### Experimental Example 7: Western blot analysis

The amount of cytoplasmic and membrane proteins was quantified by the Bradford method. The proteins were loaded on a 10% SDS-polyacrylamide gel, and electrophoresed in a Tris-glycine buffer for an hour. Thereafter, the proteins were transferred to a nylon membrane, and the positions of the bands were confirmed using a Ponceau solution. Then, the membrane was blocked with a blocking buffer (1 × TBS, 0.1% Tween-20, 5% skim milk) for 60 minutes at room temperature, and then incubated with primary antibodies overnight at 4 °C. Each of the primary antibodies was diluted in 5% skim milk as described in Table 2. After washing, the membrane was incubated in a TBST buffer (25 mM Tris-base, 155 mM NaCl, 0.1% Tween-20) for 30 minutes, and incubated with an anti-rabbit IgG (Amersham, UK) or anti-goat IgG (Abcam, USA) secondary antibody at room temperature for 1 hour. The membrane was washed with a TBST buffer for 30 minutes, an immunoreactive band was then generated using an ECL kit (Pierce Chemical Co., IL), and the molecular weight of the band was confirmed. A list of the used antibodies is shown in Table 2.

**[Table 2]**

| **Primary antibody** | **Primary antibody dilution** | **Secondary antibody** | **Secondary antibody dilution** |
|---|---|---|---|
| **GAPDH** | 1:1000 | anti-rabbit lgG | 1:10000 |
| **PGClα** | 1:1000 | anti-rabbit lgG | 1:10000 |
| **pAkt**++ | 1:1000 | anti-rabbit lgG | 1:10000 |
| **Akt** | 1:1000 | anti-rabbit lgG | 1:10000 |
| **p-PI3K** | 1:1000 | anti-rabbit lgG | 1:10000 |
| **P13K** | 1:1000 | anti-rabbit lgG | 1:10000 |
| **mTOR** | 1:1000 | anti-rabbit lgG | 1:10000 |
| **TNF-α** | 1:1000 | anti-rabbit lgG | 1:10000 |

### Experimental Example 8: Statistical analysis

Data was expressed as mean ± standard error (SE). All statistical analyses were performed using IBM SPSS Statistics 26 (SPSS, Inc., Chicago, IL, USA). The statistical difference between the YC and AC groups was analyzed using a Mann Whitney U t-test. The difference between the aging groups (AC and CTP groups) was analyzed using a Kruskal-Wallis test, and a Dunn's Multiple Comparison test was then performed. Data with other superscripts are significantly different according to a Kruskal-Wallis post-hoc test. A p-value of less than 0.05 was considered statistically significant.

### [EXAMPLES]

### Example 1: Effect of collagen on body weight, food efficiency ratio (FER) and organ weight

As shown in Table 3 (*p < 0.05, **p < 0.01, and ***p < 0.001 are YC vs AC / ##p < 0.01 is CTP vs AC; BWG: body weight gain), there was no significant difference between the aging groups (AC and CTP groups) in body weight (BW) or food intake. Feed intake and energy intake significantly decreased in the YC group compared to the AC group. On the other hand, there was no significant difference between the aging groups (AC and CTP groups). In the case of organ weights, the liver weight was higher in the AC group than in the other groups, while the kidney weight was significantly lower in the AC group than in the other groups.

**[Table 3]**

| | **YC** | **AC** | **CTP** |
|---|---|---|---|
| | Mean ± SD | Mean ± SD | Mean ± SD |
| **Initial BW (g)** | 24.70 ± 0.48 | 36.50 ± 0.47*** | 36.27 ± 0.41 |
| **Final BW (g)** | 30.06 ± 0.50 | 42.01 ± 0.85*** | 40.40 ± 0.87 |
| **Total BWG (g)** | 5.36 ± 0.24 | 5.52 ± 0.67 | 3.78 ± 0.89 |
| **Food Intake (g/day)** | 3.17 ± 0.08 | 3.50 ± 0.09* | 3.32 ± 0.08 |
| **Energy Intake (kcal/day)** | 12.53 ± 0.32 | 13.82 ± 0.34* | 13.09 ± 0.30 |
| **FER** | 0.04 ± 0.00 | 0.03 ± 0.00 | 0.03 ± 0.01 |
| **Liver (g/100g BW)** | 3.16 ± 0.05 | 3.97 ± 0.09** | 3.24 ± 0.06^{##} |
| **Kidney (g/100g BW)** | 1.00 ± 0.01 | 0.89 ± 0.01 ** | 1.02 ± 0.04^{##} |

### Example 2: Effect of collagen on sarcopenia in aged mice

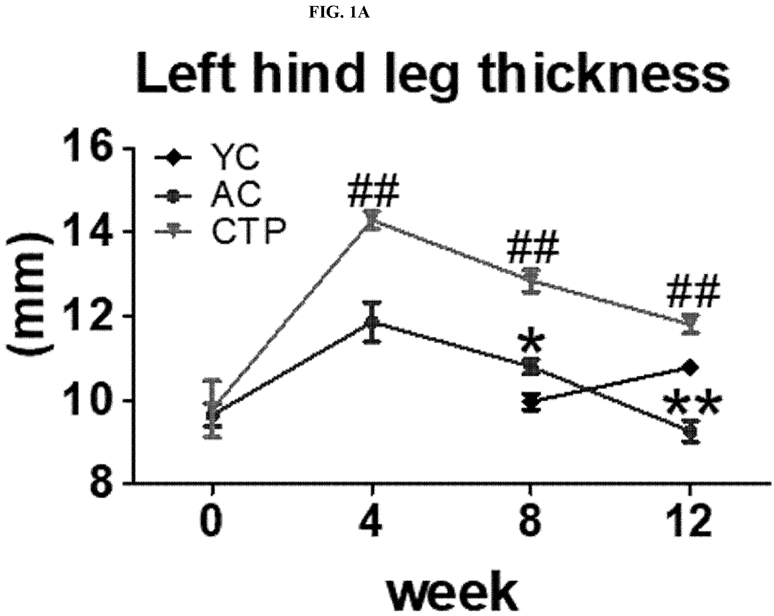

The thicknesses of the left hind legs were measured. As a result, as shown in FIG. 1A, the thicknesses of the left hind legs increased in both aging groups (AC and CTP groups) until week 4 of the experiment. Thereafter, the thicknesses of the left hind legs in the aging groups generally decreased, whereas the thicknesses of the left hind legs in the YC group increased until week 12 compared to week 8. However, it can be seen that the thicknesses of the left hind legs of the CTP group significantly increased from week 4 compared to the AC group.

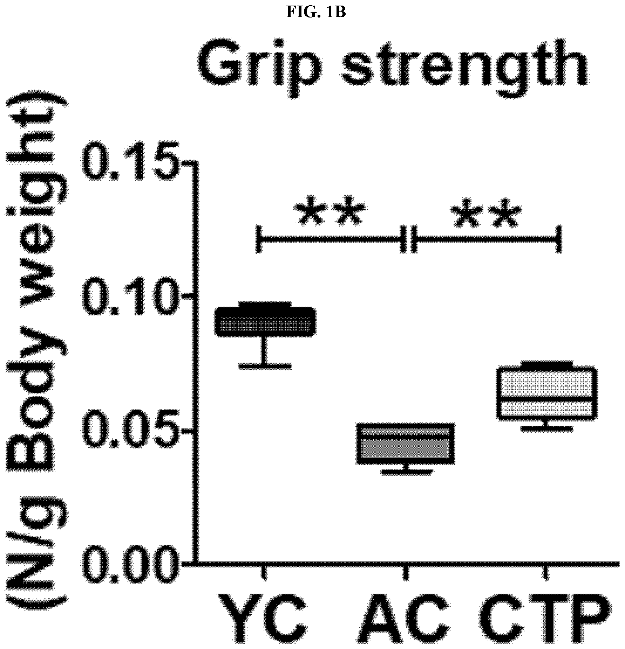

The whole-body tension was measured. As a result, as shown in FIG. 1B, it was confirmed that the whole-body tension of the AC group decreased due to aging compared to that of the YC group, but the decrease in the whole-body tension of the AC group was significantly improved by the intake of low-molecular-weight collagen.

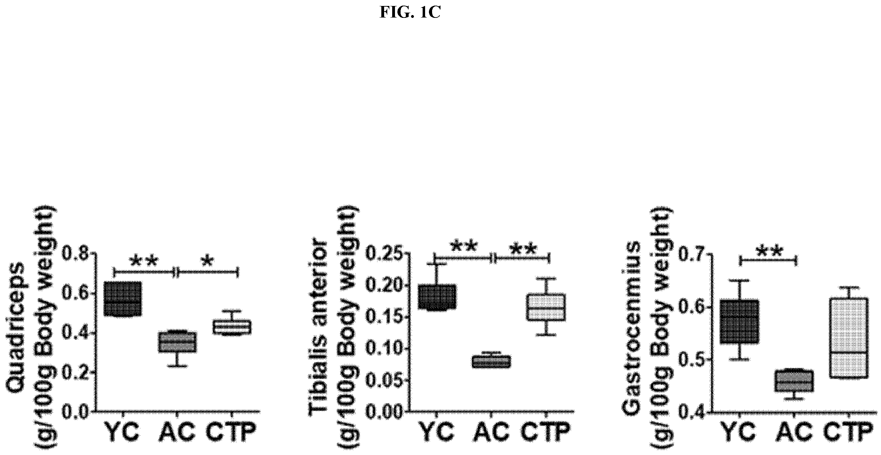

As a result of measurement of muscle weights, as shown in FIG. 1C, the weights of the quadriceps, tibialis anterior, and gastrocnemius muscles significantly decreased in the AC group compared to the YC group. However, the weights of the tibialis anterior and quadriceps were significantly higher in the CTP group compared to the AC group.

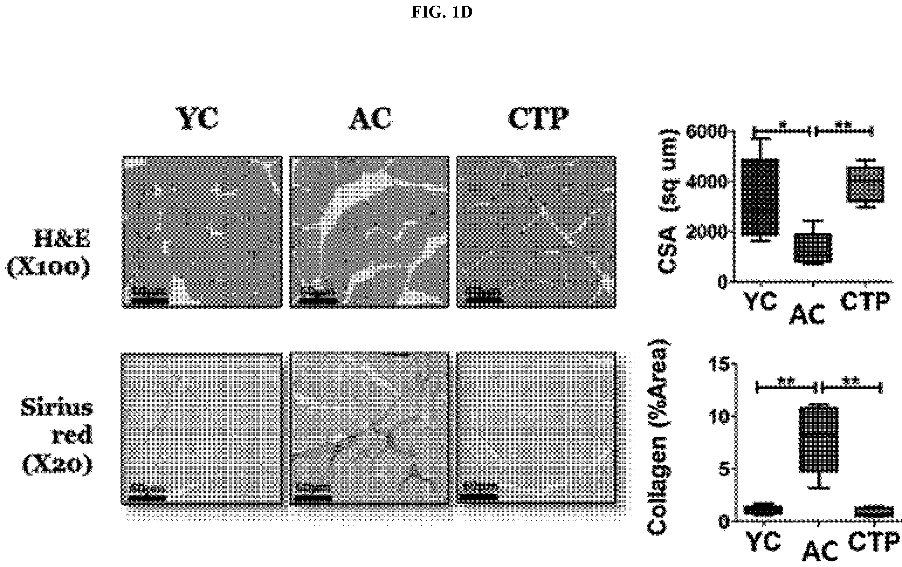

The cross-section of the gastrocnemius muscle was examined to determine whether the decrease in muscle mass actually resulted from a decrease in muscle fiber area. Accordingly, gastrocnemius muscle tissue was stained with H&E and Sirius red to evaluate the quality of the muscle tissue. As shown in FIG. 1D, it can be seen from the result of H&E staining that the AC group had smaller muscle fibers than the YC group and there were empty spaces between the muscle fibers. Also, a tendency for hypertrophic changes could be confirmed in the AC group, and a decrease in the increased collagen occupancy area could be confirmed in the CTP group.

### Example 3: Effects of collagen on protein synthesis and degradation related to expression of muscle protein

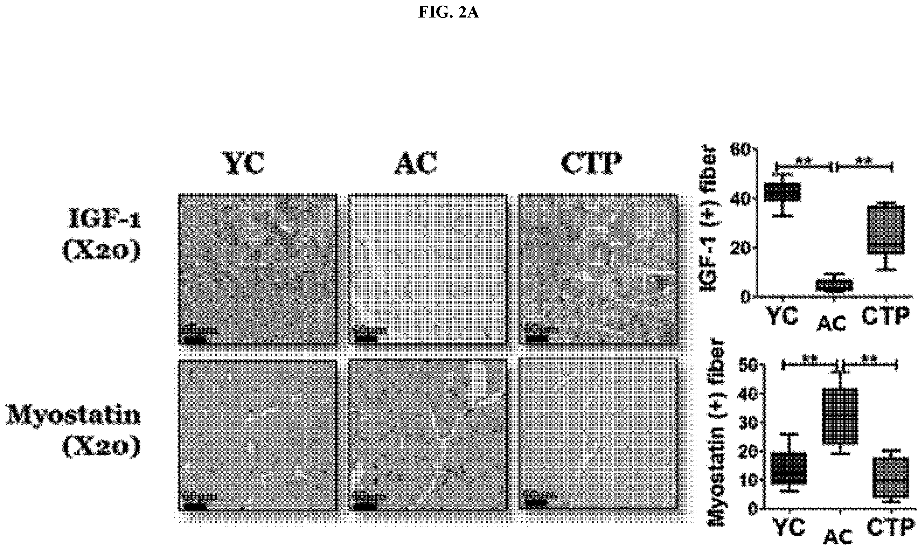

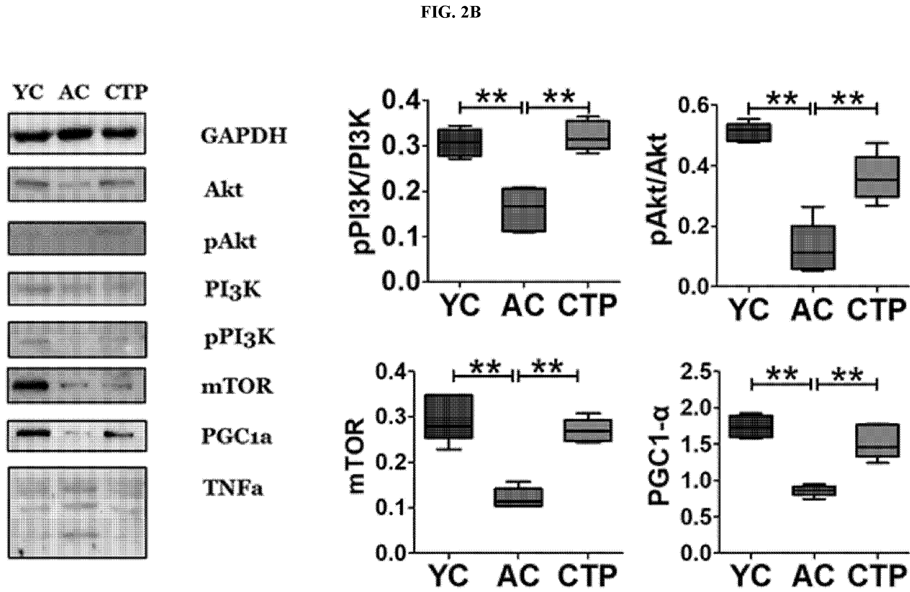

As shown in FIG. 2A, it can be seen from the immunohistochemical analysis of the gastrocnemius that the expression level of insulin-like growth factor 1 (IGF-1; an upstream factor of protein synthesis) in gastrocnemius muscle fibers significantly decreased in the AC group compared to the YC group, whereas such a decrease was suppressed by the intake of the low-molecular-weight collagen. Also, as shown in FIG. 2B, it can be seen that the expression and activation of IGF-1 downstream factors including PI3K, Akt and mTOR were changed more in the CTP group compared to the AC group, and mTOR expression significantly increased compared to the AC group.

Meanwhile, as shown in FIG. 2A, it can be seen that the expression of myostatin (an upstream factor of protein degradation) in gastrocnemius muscle fibers significantly increased in the AC group compared to the YC group, whereas such an increase was suppressed by the intake of the low-molecular-weight collagen.

### Example 4: Effect of collagen on adipose tissue weight and muscle lipid profile

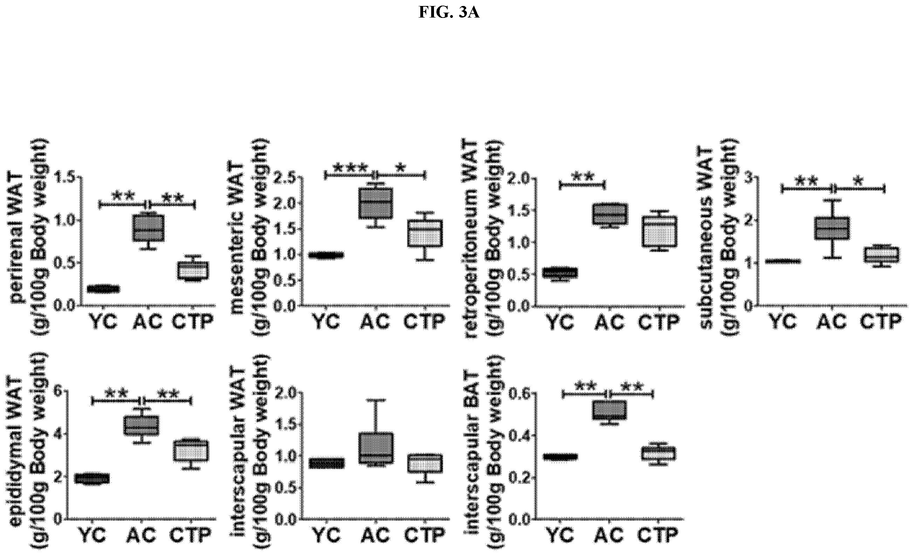

The weights of the adipose tissues in each group were measured and shown in FIG. 3A. It was found that the weights of all types of adipose tissue other than intervascular adipose tissue (not shown in FIG. 3A) increased significantly in the AC group compared to the YC group, and the increase in fat weight in the AC group with aging was suppressed by low-molecular-weight collagen supplementation.

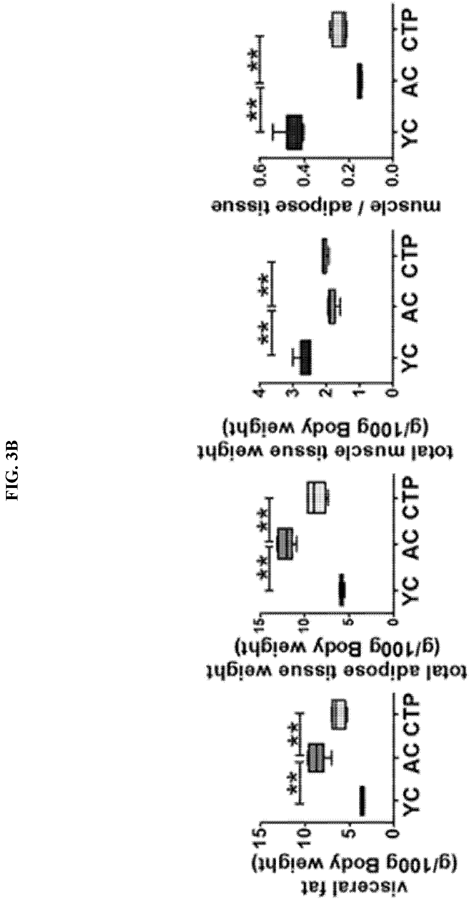

To check the effect of increasing adipose tissue weight on muscle tissue, the ratio of the total weight of muscle tissue and the total weight of adipose tissue was calculated. As a result, as shown in FIG. 3B, total muscle tissue weight decreased in the AC group compared to the YC group, but significantly increased in the CTP group compared to the AC group. Similarly, it can be seen that the muscle/fat tissue weight ratio was significantly lower in the AC group than in the YC group, and such a decrease was suppressed by low-molecular-weight collagen supplementation.

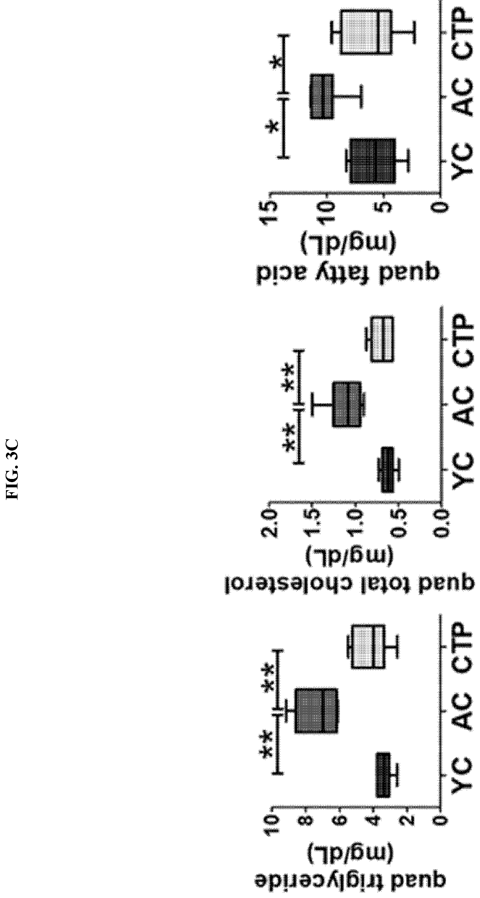

Also, the muscle lipid profile of the gastrocnemius was confirmed after 12 weeks of experimental diet feeding. As a result, as shown in FIG. 3C, it can be seen that the TG, TC and FA levels increased significantly in the AC group compared to the YC group, and such an increase in the AC group was suppressed by the intake of the low-molecular-weight collagen.

The above description of the present invention has been given by way of illustration only. Therefore, those skilled in the art to which the present invention pertains will appreciate that the present invention can be embodied in other specific forms without changing the technical spirit or essential features of the present invention. Therefore, it should be understood that the embodiments described above are for the purpose of illustration only, and not intended to be limiting in all respects.

### [Industrial Applicability]

The low-molecular-weight collagen of the present invention may inhibit the breakdown of muscle protein and exhibit a muscle-strengthening effect by increasing muscle mass. Therefore, the present invention is industrially applicable because the low-molecular-weight collagen of the present invention is expected to be effectively used in the prevention, alleviation or treatment of sarcopenia.

## Claims

1. A pharmaceutical composition for preventing or treating sarcopenia, comprising low-molecular-weight collagen as an active ingredient.

2. The pharmaceutical composition of claim 1, wherein the low-molecular-weight collagen has an average molecular weight of 1,000 daltons or less.

3. The pharmaceutical composition of claim 1, wherein the low-molecular-weight collagen comprises 2 to 10% by weight of a collagen tripeptide having a glycine-proline-hydroxyproline amino acid sequence based on the total amount of the low-molecular-weight collagen comprised in the pharmaceutical composition.

4. The pharmaceutical composition of claim 1, wherein the low-molecular-weight collagen satisfies one or more of the following characteristics:
a) increasing the expression of insulin-like growth factor 1 (IGF-1);
b) inhibiting myostatin expression;
c) inhibiting a reduction in muscle strength; and
d) increasing muscle mass.

5. The pharmaceutical composition of claim 1, wherein the pharmaceutical composition is used for people in their 30s or older who are at risk of developing sarcopenia.

6. A food composition for preventing or alleviating sarcopenia, comprising low-molecular-weight collagen as an active ingredient.

7. The food composition of claim 6, wherein the food composition is a health functional food composition.

8. The food composition of claim 6, wherein the low-molecular-weight collagen has an average molecular weight of 1,000 daltons or less.

9. The food composition of claim 6, wherein the low-molecular-weight collagen comprises 2 to 10% by weight of a collagen tripeptide having a glycine-proline-hydroxyproline amino acid sequence based on the total amount of the low-molecular-weight collagen comprised in the food composition.

10. The food composition of claim 6, wherein the low-molecular-weight collagen satisfies one or more of the following characteristics:
a) increasing the expression of insulin-like growth factor 1 (IGF-1);
b) inhibiting myostatin expression;
c) inhibiting a reduction in muscle strength; and
d) increasing muscle mass.

11. The food composition of claim 6, wherein the food composition is used for people in their 30s or older who are at risk of developing sarcopenia.

12. A feed composition for preventing or alleviating sarcopenia, comprising low-molecular-weight collagen as an active ingredient.

13. The feed composition of claim 12, wherein the low-molecular-weight collagen has an average molecular weight of 1,000 daltons or less.

14. A method of preventing or treating sarcopenia, comprising:
administering a composition comprising low-molecular-weight collagen as an active ingredient to a subject in need thereof.

15. A use of a composition comprising low-molecular-weight collagen as an active ingredient for the prevention or treatment of sarcopenia.

16. A use of low-molecular-weight collagen for the manufacture of a drug for the treatment of sarcopenia.
